# EUROPEAN PATENT APPLICATION

(11) **EP 1 774 961 A2**
(43) Date of publication of application: **18.04.2007**
(21) Application number: 06255045.4
(22) Date of filing: 29.09.2006
(51) Int. Cl.: A61K 9/08, A61K 47/32, A61K 31/4535, A61K 31/55, A61K 31/335

(54) **Methods for stabilizing ophthalmic compositions**

(30) Priority: 30.09.2005 US 240930
(71) Applicant: Johnson and Johnson Vision Care, Inc., Jacksonville, FL 32256 (US)
(72) Inventor: Alli, Azaam, Jacksonville, FL 32277 (US); Mahadevan, Shivkumar, Orange Park, FL 32003 (US)
(74) Representative: Fisher, Adrian John

(57) **Abstract**

The present invention relates to a method comprising stabilizing, during autoclaving, an oxidatively unstable ophthalmic compound dissolved in an ophthalmic solution by incorporating a stabilizing effective amount of at least one electron rich polymer in said ophthalmic solution.

## Description

### FIELD OF THE INVENTION

The present invention relates to methods for stabilizing ophthalmic against oxidative degradation, during processing, autoclaving, packaging, shipping or storage.

### BACKGROUND OF THE INVENTION

Therapeutic agents for topical administration to the eye are generally formulated in either a liquid or gel form and must be kept sterile until administration. Accordingly, ophthalmic therapeutic agents are either packaged asceptically, which is cumbersome and expensive or are heat sterilized. Unfortunately, many therapeutic agents are not oxidatively stable, especially at elevated temperatures.

EDTA has been used to improve the stability of certain therapeutic agents during autoclaving. However, there remains a need for other compounds capable of stabilizing unstable therapeutic agents that are susceptible to catalytic oxidative degradation.

### SUMMARY OF THE INVENTION

The present invention relates to a method comprising stabilizing, during autoclaving, an oxidatively unstable ophthalmic compound dissolved in an ophthalmic solution by incorporating a stabilizing effective amount of at least one electron rich polymer in said ophthalmic solution.

### DETAILED DESCRIPTION OF THE INVENTION

The present invention comprises, consists of and consists essentially of stabilizing, during autoclaving, at least one oxidatively unstable ophthalmic compound dissolved in an ophthalmic solution by incorporating a stabilizing effective amount of at least one electron rich polymer in said ophthalmic solution.

As used herein, oxidatively unstable ophthalmic compound ("OUOC") is any therapeutic agent which shows greater than 10% degradation when autoclaved in solution with at least one oxidative catalyst, but shows less than 10% degradation when autoclaved under the same conditions without said at least one oxidative catalyst. Oxidative instability may be measured by forming a solution of 3 ml packing solution containing 25 ppm of the therapeutic agent to be evaluated, and exposing the solution, with and without oxidative catalysts (100 ppm Cu₂O and 100 ppm FeSO₄) to autoclave conditions (120°C for 20 minutes).

Examples of OUOC include oxidatively unstable pharmaceutical and nutraceutical compounds. In one embodiment the OUOC comprises at least one pharmaceutically active amine. In one embodiment the OUOC comprises at least one tertiary cyclic amine. In another embodiment the OUOC comprises at least one tertiary cyclohexyl amine. In another embodiment the OUOC comprises at least one therapeutic agent selected from acycylovir, adrenalone, aminocaproic acid, amoxicillin, amotriphene, amoxecaine, amodiaquin, antazoline, atrophine, betaxolol, bupivacaine, carbachol, carteolol, chlorampenicol, chlortetracycline, corynathine, cromalyn sodium, cyclopentolate, demecarium, dexamethasone, dichlorphenamide, dibutoline, diclophenac, dipivefrin, ephedrine, erythromycin, ethambutol, eucatropine, fluoromethalone, gentamycin, gramicidin, homatropine, indomethacin, ketotifen, levallorphan, levobunolol, levocabastine, lidocaine, lignocaine, lomefloxacin, medrysone, mepivacaine, methazolamide, naphazoline, natamycin, natamycin, neomycin, noradrenaline, ofloxacin, oxybuprocaine, oxymetazoline, pheniramine, phenylephrine, physostigmine, pilocarpine, polymyxin B, prednisolone, proparacaine, pyrilamine, scopolamine, sorbinil, sulfacetamide, tamoxifen, tetracaine, tetracycline, tetrahydozoline, timolol, trifluridine, tropicamide, vidarabine, and salts and mixtures thereof Examples of nutriceutical compounds include vitamins and supplements such as vitamins A, D, E, lutein, zeaxanthin, lipoic acid, flavonoids, ophthalmicially compatible fatty acids, such as omega 3 and omega 6 fatty acids, combinations thereof, combinations with pharmaceutical compounds and the like. In yet another embodiment the OUOC comprises at least one therapeutic agent selected from ketotifen fumarate, nor ketotifen fumarate, 11-dihydro-11-(1-methyl-4-piperidinylidene)-5H-imidazo[2,1-b][3]benzazepine-3-carboxaldehyde (CAS# 147084-10-4), olapatadine and mixtures thereof. In yet another embodiment the OUOC comprises at least one therapeutic agent selected from ketotifen fumarate, 11-dihydro-11-(1-methyl-4-piperidinylidene)-5H-imidazo[2,1-b][3]benzazepine-3-carboxaldehyde (CAS# 147084-10-4) and mixtures thereof.

The concentration of the OUOC in the oxidatively stable ophthalmic compositions of the present invention may range from about 10 ppm to about 100,000 ppm, in some embodiments from about 10 to about 10,000 ppm, in some embodiments from about 10 to about 1,000 ppm and some embodiments from about 10 to about 500 ppm.

The oxidatively stable ophthalmic compositions of the present invention further comprise at least one electron rich polymer. Suitable electron rich polymers are water-soluble, comprise at least one group with a free electron pair, have a weight average molecular weight, Mw, greater than about 1,000 and in some embodiments between about 1000 and about 2,000,000, and are substantially free from transition metal containing species. As used herein, water soluble means that the selected electron rich polymer does not precipitate or form visible gel particles at the concentrations selected and across the temperatures and pH regimes common for manufacturing, sterilizing and storing ophthalmic solutions. In some embodiments the electron rich polymer is substantially free from copper and iron containing species.

For purposes of the invention, the molecular weight is determined using a gel permeation chromatography with a 90° light scattering and refractive index detectors. Two columns of PW4000 and PW2500, a methanol-water eluent of 75/25 wt/wt adjusted to 50mM sodium chloride and a mixture of polyethylene glycol and polyethylene oxide molecules with well defined molecular weights ranging from 325,000 to 194 are used.

As used herein, "substantially free from" means that transition metal containing species are present in the electron rich polymer in amounts which are insufficient to cause further degradation of the OUOC. Preferably transition metal containing species are present in the electron rich polymer in amounts less than about 100 ppm, in some embodiments less than about 50 ppm and in some embodiments less than about 20 ppm.

Suitable electron rich polymers include polymers comprising esters, acids, amines, carbonates, carboxylates, thiols, lactates, amides, carbamates, phosphates, phosphines, nitriles, lactams, and combinations thereof Polymers which do not have groups with at least one free electron pair, such as polymers comprising only ether groups, alcohol groups or combinations thereof are not electron rich polymers are defined herein. A wide concentration of electronic donating groups may be included, however, the higher the concentration of electron donating groups, the less electron rich polymer will need to be used. Specific examples include homopolymers and random or block copolymers of methacrylic acid, acrylic acid, itaconic acid, fumaric acid, maleic acid, vinylpyrollidone, vinylmethacetimide, combinations thereof and the like. More specific examples include poly(acrylic acid), poly(vinylpyrollidone) and poly(vinylmethylacetamide) and combinations thereof and the like. In one embodiment the electron rich polymer is poly(acrylic acid).

The electron rich polymer is present in the ophthalmic composition in stabilizing effective amounts. A stabilizing effective amount will vary depending upon the OUOC, the concentration of the OUOC and the concentration of other components in the ophthalmic composition, but generally stabilizing effective amounts are those sufficient to provide at least about a 5% improvement in stability. Suitable amounts of electron rich polymer include between about 10 and about 5,000 ppm, in some embodiments between about 100 and about 5,000 ppm, in some embodiments between about 500 and about 3,000 ppm.

The OUOC and electron rich polymer may be combined in any suitable ophthalmically compatible carrier. Suitable carriers include water, saline solution, mineral oil, petroleum jelly, water soluble solvents, such as C₁₅₋₂₀ alcohols, C₁₅₋₂₀ amides, C₁₅₋₂₀ alcohols substituted with zwitterions, vegetable oils or mineral oils comprising from 0.5 to 5% by weight hydroxyethylcellulose, ethyl oleate, carboxymethylcellulose, polyvinyl-pyrrolidone and other non-toxic water-soluble polymers for ophthalmic uses, such as, for example cellulose derivatives, such as methylcellulose, alkali metal salts of carboxy-methylcellulose, hydroxymethylcellulose, hydroxyethylcellulose, methylhydroxypropyl-cellulose, hydroxypropylcellulose, chitosan and scleroglucan, acrylates or methacrylates, such as salts of poly(acrylic acid) or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as poloxamers, e.g. Poloxamer F127, polyvinyl alcohol, polyvinylpyrrolidone, polyvinyl methyl ether, polyethylene oxide, preferably cross-linked poly(acrylic acid), such as neutral Carbopol, or mixtures of those polymers. Preferred carriers are water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol, or mixtures thereof The concentration of the carrier is, for example, from 0.1 to 100000 times the concentration of the active ingredient combinations thereof and the like. When the ophthalmic composition is an eye drop, preferred carriers include water, pH buffered saline solution, mixtures thereof and the like.

The oxidatively stable compositions of the present invention may also be used as the packaging or storage solution for an ophthalmic device, such as a contact lens. When the ophthalmic composition of the present invention is used as a packaging solution for a contact lens the carrier comprises a buffered saline solution. Any contact lens could be packaged with the oxidatively stable ophthalmic compositions of the present invention, including, but not limited to commercially available hydrogel formulations such as etafilcon, polymacon, vifilcon, genfilcon A, lenefilcon A, galyfilcon, senofilcon, balafilcon, lotrafilcon A, lotrafilcon B and the like.

The compositions of the present invention may further comprise additional components such as antioxidants, demulcents, antibacterial agents, solubilizers, surfactants, buffer agents, tonicity adjusting agents, chelating agents, preservatives, wetting agents, thickeners, combinations thereof and the like. The oxidatively stable compositions of the present invention must be ophthalmically compatible. If the addition of the electron rich polymer decreases the pH of the ophthalmic composition to an undesirable level, a stoichometric amount of a base, such as sodium hydroxide, or a buffering agent, such as sodium borate, may be added. Generally a pH between about 5 and about 9, in some embodiments between about 6 to about 8 is desired. In some embodiments, the stabilizing effect of the electron rich polymer may be most effective at a pH of about 6.5 to about 7.5.

The oxidatively stable compositions of the present invention may be formed by mixing the OUOC and the electron rich polymer with the selected carrier. When a liquid composition, such as an eye drop or packaging solution for a contact lens, the OUOC and the electron rich polymer are dissolved in the carrier. When the oxidatively stable composition is a gel or an ointment, the OUOC and electron rich polymer may be incorporated in any suitable manner, such as dissolved, mixed or compounded into the selected carrier.

It is generally desirable that the shelf life of the oxidatively stable compositions of the present invention be in excess of about 6 months, and in some instances greater than about 1 year, or even more than about 2 years. During the shelf life of the oxidatively stable compositions it is desirable that at least about than 80% of the original concentration of the OUOC remains, and in some embodiments greater than about 90%.

These examples do not limit the invention. They are meant only to suggest a method of practicing the invention. Those knowledgeable in contact lenses as well as other specialties may find other methods of practicing the invention. However, those methods are deemed to be within the scope of this invention.

### Examples 1 -3.

The solutions of Table 1 were formed by combining the listed components with reverse osmosis purified water to a total volume of 1 liter. The pH of these solutions was adjusted to 7.5, 7.3, and 6.9 with addition of HCl. To each solution was added 80 ppm of ketotifen fumarate. 3.0 ml of each of these solutions were placed in a number of vials. Each vial was autoclaved, heating to 121°C and holding at that temperature for 30 minutes, for the number of cycles indicated in Table 2, using three vials per each set of conditions. The concentration of ketotifen was determined using HPLC using an HP 1100 with an Agilent Zorbax Eclipse XDB-C18 and Rapid Resolution HT 50 x 4.6 mm x 1.8 µ column and the following conditions:
Detector Wavelength : 299 nm
Flow rate: 1.0 mL/min
Injection Volume: 3 µL
Mobile Phase:
- Eluent A:: 17 % acetonitrile in 0.025 M dihydrogen potassium phosphate buffer
0.2 % triethylamine, 0.13 % o-phosphoric acid
- Eluent B:: 50 % acetonitrile in 0.025 M dihydrogen potassium phosphate buffer
0.2 % triethylamine, 0.13 % o-phosphoric acid

| Time (min) | Eluent A (%) | Eluent B (%) |
|---|---|---|
| 0 | 100 | 0 |
| 5 | 100 | 0 |
| 20 | 0 | 100 |
| 21 | 100 | 0 |
| 25 | 100 | 0 |

Table 2 shows the average of three measurements of the concentration of ketotifen remaining in the vials as a percentage of the original concentration.

**Table 1**

| Comp (gm/100 ml) | Ex.1 | Ex.2 | Ex. 3 | CEx 1 | CEx 2 | CEx 3 |
|---|---|---|---|---|---|---|
| NaCl | 0.83 | 0.83 | 0.83 | 0.83 | 0.83 | 0.83 |
| Boric acid | 0.91 | 0.91 | 0.91 | 0.91 | 0.91 | 0.91 |
| Sodium borate | 0.1 | 0.1 | 0.1 | 0.1 | 0.1 | 0.1 |
| PAA (MW = 225,000) | 0.1 | 0.1 | 0.1 | 0 | 0 | 0 |

**Table 2 - Ketotifen concentration after autoclaving**

| | Ex.1 | Ex.2 | Ex.3 | CEx 1 | CEx 2 | CEx 3 |
|---|---|---|---|---|---|---|
| PH | 7.5 | 7.3 | 6.9 | 7.5 | 7.3 | 6.9 |
| 0 cycle | 100 | 100 | 100 | 100 | 100 | 100 |
| 1 cycle | 64 | 96 | 96 | 0 | 0 | 0 |
| 2 cycle | 55 | 86 | 84 | - | - | - |
| 3 cycle | 22 | 64 | 62 | - | - | - |

### Examples 4-6

The solution of Example 1 was made but with the amounts of PAA shown in Table 3, below. The pH of each solution was adjusted to 7.22 using 0.1 N HCl. As in Example 1 80 ppm (wt) ketotifen fumarate added to each solution. The stability of the solutions was tested as in Example 1. The results are shown in Table 3.

**Table 3**

| | Ex. 4 | Ex. 5 | Ex. 6 |
|---|---|---|---|
| [PAA] ppm | 500 | 2000 | 5000 |
| O cycles | 100 | 100 | 100 |
| 1 cycle | 88 | 94 | 94 |
| 2 cycles | 64 | 81 | 83 |
| 3 cycles | 36 | 66 | 70 |

### Example 7

A ketotifen fumarate solution was formed by combining the components listed in Table 4 with reverse osmosis purified water to a total volume of 1 liter. The pH of the solution was about 7.2. 3.0 ml of the solution was placed in each of three vials. The initial concentration of ketotifen was determined using HPLC. The vials were autoclaved, heating to 121°C and holding at that temperature for 30 minutes. The concentration of ketotifen remaining after one autoclave cycle was determined using HPLC as described above.

The average concentration of ketotifen furmarate remaining was 80 µg/ml. Accordingly, only about 17% of the ketotifen fumarate was lost during a single autoclave cycle. A control solution, having the components in Table 4, but without the 400 ppm PVP lost over 50% of the ketotifen fumarate during a single autoclave cycle.

**Table 4**

| Component | |
|---|---|
| NaCl | 0.83 gm/ml |
| Boric acid | 0.91 gm/ml |
| Sodium borate | 0.1 gm/ml |
| PVP (MW = 90,000) | 400 ppm |
| Ketotifen fumarate | 97 µg/ml |

Thus the foregoing examples clearly show that the inclusion of at least one electron rich polymer, such as poly(acrylic acid), significantly improves the stability of an oxidatively unstable ophthalmic composition, like ketotifen fumarate.

## Claims

1. A method comprising stabilizing, during autoclaving, an oxidatively unstable ophthalmic compound dissolved in an ophthalmic solution by incorporating a stabilizing effective amount of at least one electron rich polymer in said ophthalmic solution.

2. An autoclavable solution comprising an ophthalmic solution comprising at least one oxidative unstable ophthalmic compound and a stabilizing effective amount of at least one electron rich polymer.

3. The method of claim 1 or the autoclavable solution of claim 2, wherein said oxidative unstable ophthalmic compound is at least one pharmaceutically active amine.

4. The method or autoclavable solution of claim 3, wherein said pharmaceutically active amine is a tertiary cyclic amine.

5. The method or autoclavable solution of claim 3, wherein said pharmaceutically active amine is a tertiary cyclohexyl amine.

6. The method of claim 1 or the autoclavable solution of claim 2, wherein said oxidatively unstable ophthalmic compound is selected from the group consisting of ketotifen fumarate, nor ketotifen fumarate, 11-dihydro-11-(1-methyl-4-piperidinylidene)-5H-imidazo[2,1-b][3]benzazepine-3-carboxaldehyde, olapatadine and mixtures thereof.

7. The method of claim 1 or the autoclavable solution of claim 2, wherein said oxidatively unstable ophthalmic compound is selected from the group consisting of ketotifen fumarate, 11-dihydro-11-(1-methyl-4-piperidinylidene)-5H-imidazo[2,1-b][3]benzazepine-3-carboxaldehyde, and mixtures thereof

8. The method of claim 1 or the autoclavable solution of claim 2, wherein said oxidatively unstable ophthalmic compound comprises ketotifen fumarate.

9. The method or autoclavable solution of any one of claims 1 to 8, wherein said electron rich polymer is selected from the group consisting of polyethers, polyesters, polyacids, polyamines, polycarbonates, polycarboxylates, polythiols, polylactates, polyamides, polycarbamates, polyphosphates, polynitriles, polylactams, and copolymers and mixtures thereof.

10. The method or autoclavable solution of any one of claims 1 to 8, wherein said electron rich polymer is selected from the group consisting poly(acrylic acid), poly(vinylpyrollidone) and poly(vinylmethylacetamide), and mixtures thereof.

11. The method or autoclavable solution of any preceding claim, wherein said electron rich polymer is present in an amount between 10 and 5000 ppm.

12. The method or autoclavable solution of claim 11, wherein said electron rich polymer is present in an amount between 100 and 5000 ppm.
